(19) Europäisches Patentamt
European Patent Office
Office européen des brevets

(11) EP 2 079 845 B1

(12) **EUROPEAN PATENT SPECIFICATION**

(45) Date of publication and mention
of the grant of the patent:
**21.05.2014 Bulletin 2014/21**

(51) Int Cl.:
*C12P 19/04* (2006.01)       *C08L 1/02* (2006.01)
*A61L 27/20* (2006.01)       *A61L 27/38* (2006.01)
*A61L 27/50* (2006.01)

(21) Application number: **07820833.7**

(22) Date of filing: **02.10.2007**

(86) International application number:
**PCT/EP2007/060451**

(87) International publication number:
**WO 2008/040729 (10.04.2008 Gazette 2008/15)**

(54) **Process for producing vessels containing bacterial cellulose**

Verfahren zur Herstellung von bakterielle Cellulose enthaltenden Gefässen

Procédé de production de vaisseaux comprenant de cellulose bactérienne

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.10.2006 SE 0602110**

(43) Date of publication of application:
**22.07.2009 Bulletin 2009/30**

(60) Divisional application:
**11188474.8 / 2 426 211**

(73) Proprietor: **Arterion AB**
**413 46 Gothenburg (SE)**

(72) Inventors:
• **BODIN, Aase**
**431 39 Mölndal (SE)**
• **BÄCKDAHL, Henrik**
**412 59 Gothenburg (SE)**
• **GATENHOLM, Paul**
**429 35 Kullavik (SE)**
• **GUSTAFSSON, Lena**
**101 58 Stockholm (SE)**
• **RISBERG, Bo**
**452 95 Strömstad (SE)**

(74) Representative: **Jörgensson, Leif Sixten**
**Ström & Gulliksson AB**
**P.O. Box 4188**
**203 13 Malmö (SE)**

(56) References cited:
**WO-A-2007/027849**

• **BODIN, A. ET AL.: "Manufacturing and characterization of bacterial cellulose tubes using two different fermentation techniques" MODERN MULTIDISCIPLINARY APPLIED MICROBIOLOGY (MENDEZ-VILAS, A. (ED.)), WILEY-VCH, WEINHEIM, DE, July 2006 (2006-07), pages 619-622, XP008086910**
• **KLEMM, D. ET AL.: "Bacterial synthesized cellulose - artificial blood vessels for microsurgery" PROGRESS IN POLYMER SCIENCE, vol. 26, no. 9, 2001, pages 1561-1603, XP002438400 cited in the application**
• **HULT, E.L. ET AL.: "Aggregation of ribbons in bacterial cellulose induced by high pressure incubation" CARBOHYDRATE POLYMERS, vol. 53, no. 1, July 2003 (2003-07), pages 9-14, XP004462904**
• **BODIN, A. ET AL.: "Influence of cultivation conditions on mechanical and morphological properties of bacterial cellulose tubes" BIOTECHNOLOGY AND BIOENGINEERING, vol. 97, no. 2, 1 June 2007 (2007-06-01), pages 425-434, XP002446749**

**Description**

FIELD OF THE INVENTION

The present invention relates to an improved method for the preparation of hollow cellulose vessels produced by a microorganism. Hollow cellulose vessels prepared by this method are also disclosed. The hollow microbial cellulose vessels can be used as a substitute for an internal hollow organ such as the ureter, the trachea, a digestive tract, a lymphatic vessel or a blood vessel.

BACKGROUND OF THE INVENTION

It is well known from e.g. JP 3 165 774 A1 to use cellulose produced by a microorganism (hereinafter referred to as "microbial cellulose") as biomaterial in surgical applications, such as tissue implants, for example, for the abdominal wall, the skin, subcutaneous tissue, organs, for the digestive tract, for the oesophagus, the trachea, and the urethra, as well as for cartilaginous tissue and for lipoplastics. Furthermore, it is known (for example, from JP 8 126 697 A2, EP 186 495 A2, JP 63 205 109 A1, JP 3 165 774 A1) that the microbial cellulose can be specifically shaped for its respective application in its production process, for example, in the shape of lamina, rods, cylinders and strips etc.

Furthermore, it is known from JP 3 272 772 A2 and EP 396 344 A2 to use shaped biomaterial as micro-luminal blood vessel substitutes, whereby the vessel prosthesis is cultivated on a hollow support which is permeable to oxygen (for example cellophane, Teflon, silicon, ceramic material, non-woven texture, fibres). The described process for producing the hollow microbial cellulose comprises the culturing of a cellulose synthesizing microorganism on the inner and/or outer surface of a hollow support permeable to oxygen, said support being made of cellophane, Teflon, silicon, ceramic material, or of a non-woven and woven material, respectively. Said hollow support permeable to oxygen is inserted into a culture solution. A cellulose synthesizing microorganism and a culture medium are added to the inner side and/or to the outer side of the hollow support. The culturing takes place under addition of an oxygenous gas (or liquid) also to said inner side and/or to the outer side of the hollow support. A gelatinous cellulose of a thickness of 0.01 to 20 mm forms on the surface of the hollow support.

Another process for producing the hollow microbial cellulose described in EP 396 344 A2 is the manufacturing by way of two glass tubes of different diameter. The glass tubes are inserted into one another and the culturing of the microorganisms is carried out in the space between the two tube walls within 30 days. The result is microbial cellulose of a hollow cylindrical shape which was evaluated for its blood compatibility, antithrombogenic property by a blood vessel substitute test in a dog. Parts of the descending aorta and of the jugular vein of the dog were replaced by the artificial blood vessel having an inner diameter of 2-3 mm. After one month the artificial blood vessel was removed and examined as to the state of the adhesion of clots. There was deposition of clots in the range of the suture and a slight adhesion of clots was observed over the entire inner surface of the artificial blood vessel.

WO 01/610 26 A1 and (Klemm et al. Prog. Polymer Sci. 26 (2001) 1561-1603) describe a method for producing shaped biomaterial by means of culturing cellulose producing bacteria in a cylindrical glass matrix, in particular for microsurgical applications as blood vessel substitutes of 1-3 mm diameter and smaller. This method produces microbial cellulose with a horizontal layered structure which is less suited as substitutes for larger blood vessels due to inferior mechanical properties, e.g. low burst pressure.

WO 89/12107 describes various methods for producing microbial cellulose at a gas/liquid interface. It is suggested that the yield of cellulose can be improved by increasing the concentration of oxygen available to the bacteria by bubbling, agitation or increasing the pressure or concentration of oxygen in the ambient gas environment.

US 6,017,740 and the corresponding EP 0792935 describe a process for the production of bacterial cellulose in an aerated and agitated fermentation tank. Use of increased oxygen pressure and content are suggested to increase the yield of microbial cellulose.

WO 2007/027849 discloses compositions and methods for the integration of a non- allergenic nano-cellulose into a wound bed. The composition may be formed into a wide variety of implants, e.g., a suture, a sheet, a compress, a bandage, a band, a prosthesis, a fiber, a woven fiber, a bead, a strip, a clasp, a prosthesis, a catheter, a screw, a bone plate, a pin, a bandage or combinations thereof.

The document BODIN, A. ET AL.: "Manufacturing and characterization of bacterial cellulose tubes using two different fermentation techniques" (MODERN MULTIDISCIPLINARY APPLIED MICROBIOLOGY (MENDEZ-VILAS, A. (ED.)), WILEY-VCH, July 2006, pages 619-622) discloses a method for producing hollow cellulose vessels by culturing cellulose-producing microorganisms on the outer surface of a hollow carrier and providing an oxygen containing gas on the inner side of the hollow carrier, characterized by the oxygen containing gas being air having having an oxygen level of 21 %

SUMMARY OF THE INVENTION

The object of the present invention is to provide an improved method for the preparation of hollow microbial cellulose vessels which permits for reproducible preparation of hollow microbial cellulose vessels by means of culturing cellulose-producing microorganisms on the outer surface of a hollow carrier. The method is characterized by the culturing being performed by supplying a gas with an oxygen level in the range 35 % to 100 % on the inner side of the hollow carrier. The resulting microbial cellulose vessels are characterized by a high mechanical resistance, high burst pressure, high penetration resistance.

By the method of the invention there is provided a hollow cellulose vessel comprising cellulose produced by a microorganism. The hollow microbial cellulose vessel is obtained by culturing a cellulose-producing microorganism on the outer surface of a hollow carrier composed of a non-porous material characterized by a high oxygen permeability.

Oxygen is reported to be a limiting factor for the yield of microbial cellulose produced (Schramm & Hestrin J. Gen. Microbiol. 11 (1954) 123-129. On the other hand Watanabe et al. (Biosci. Biotechnol. Biochem. 59 (1995) 65-68) reported that a higher oxygen tension in the gaseous phase than atmospheric air inhibits bacterial cellulose production and results in a denser network of the produced cellulose membrane.

The present inventors have designed a new method for the production of hollow microbial cellulose vessels which is characterized by continuous supply of appropriate levels of oxygen to the cellulose producing micoorganisms which allows for the production of microbial cellulose with superior mechanical properties. The continuous supply of appropriate levels of oxygen to the cellulose producing micoorganisms, as provided by the method of the present invention, not only increases the yield of cellulose, but also more importantly results in the production of microbial cellulose vessels with improved mechanical properties. This is evident from the significant increase in burst pressure of microbial cellulose tubes produced according to the invention at increasing oxygen ratios as further described in Example 2.

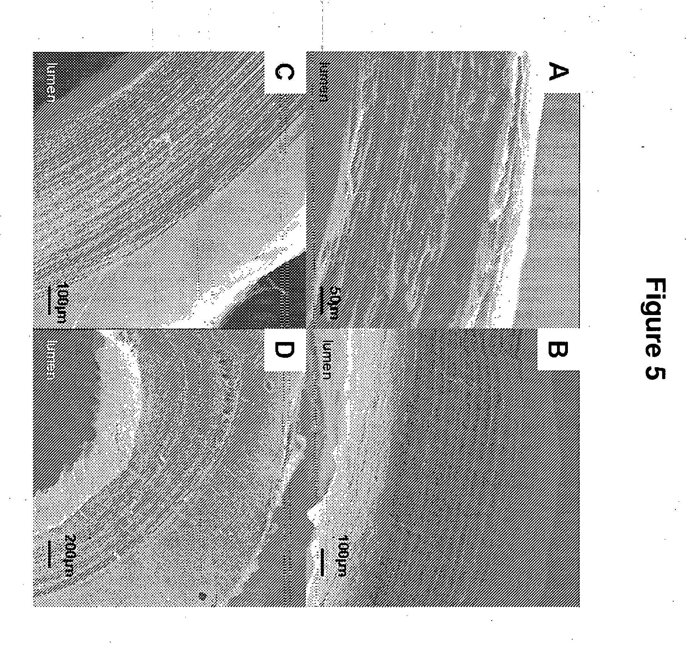

Most importantly the method of the present invention provides hollow microbial cellulose vessels where the cellulose is layered in parallel to the wall of the vessel (as shown in Example 1 and Figure 5) and where the inner layer has high density giving a high penetration resistance (as demostrated in Example 2).

Consequently, the microbial cellulose vessels produced according to the present invention have superior mechanical properties compared to microbial cellulose vessels produced according to methods described in the art.

The method of the present invention further enables a shorter cultivation period as compared to previously described methods.

E.g., the method described by Klemm et al. (WO 01/610 26 A1, Prog. Polymer Sci. 26 (2001) 1561-1603) results in microbial cellulose vessels with the cellulose layered perpendicular to the wall of the vessel giving vessels with inferior mechanical properties. The cultivation period used in this method is 14 days.

The produced hollow microbial cellulose vessels can be of any dimension, linear, tapered and/or branched.

Cultivation of endothelial cells onto the lumen of the bacterial cellulose tubes shows that a confluent layer of endothelial cells is formed after 7 days (Example 3) evidencing a very good bio-compatibility and suitability of the microbial cellulose vessels and tubes for the use in biomedical and cardiovascular applications, in particular as artificial vessels, such as artificial blood vessels. The vessels and tubes can also be cut open and the so formed patches used as patches to repair natice vessels in e.g. cardiovascular applications.

DETAILED DESCRIPTION OF THE INVENTION

The object of the present invention is an improved method for the preparation of hollow microbial cellulose vessels by means of culturing cellulose-producing microorganisms on the outer surface of a hollow carrier. An oxygen containing gas is provided on the inner side of the hollow carrier.

The method is further characterized by the oxygen containing gas having an oxygen level higher than atmospheric oxygen. The culturing is performed at an oxygen level in the range 35 % to 100 %, in the range 50 % to 100 %, in the range 60 % to 100 %, in the range 70 % to 100 %, in the range 80 % to 100 %, or in the range 90 % to 100 %. The remaining part of the gas used can be any inert gas, such as nitrogen, argon, helium. The percentage oxygen is given as v/v percentage.

Preferably the culturing is performed at an oxygen level of 100 %.

To further increase the partial pressure of oxygen, the gas can be provided at a pressure higher than atmospheric pressure, e.g. at a pressure of more than 0.2, 0.5, 1.0, 2.0 or 5.0 bar above atmospheric pressure.

Preferably, the method is performed by culturing cellulose-producing microorganisms on a hollow carrier which is positioned at a vertical position in the culture media.

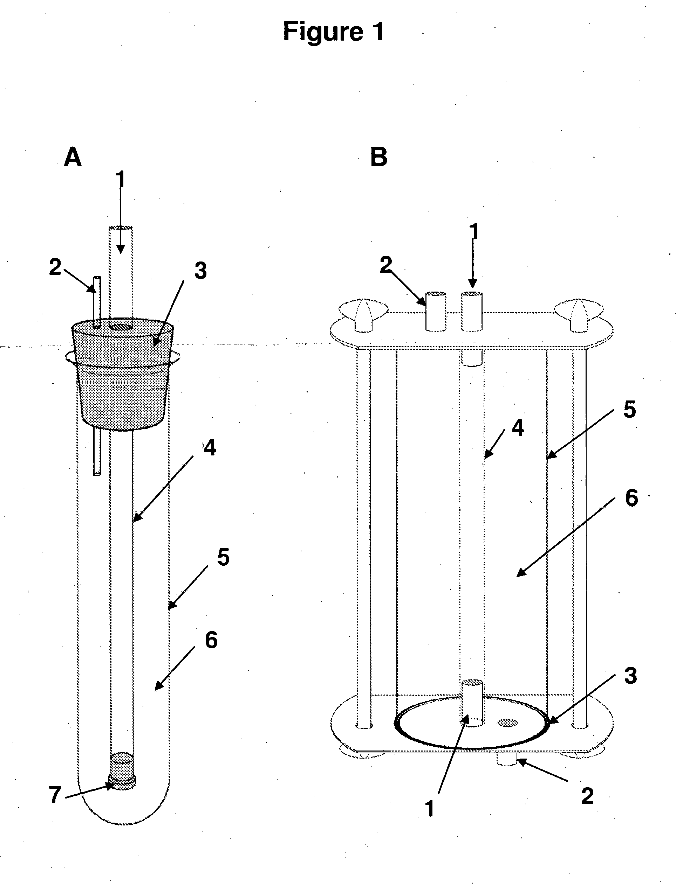

The oxygen containing gas can be provided from the top of the hollow carrier, from the bottom of the hollow carrier, or simultaneously both from the top and the bottom of the hollow carrier. Examples of suitable fermentation vessels are outlined in Figure 1.

The method for the production of microbial cellulose vessels according to the present invention is further characterized by a cultivation period of less than 10 days, such as less than 7 days, or a cultivation period of 5 days or less.

Preferably, the method is performed by culturing cellulose-producing microorganisms on a hollow carrier which is composed of a non-porous material permeable to oxygen, preferably the material has a high oxygen permeability.

Permeability (P) is the product of diffusivity (D) and solubility (S) coefficients.

$$P_i = \overline{D_i} \times \overline{S_i}$$

Oxygen permeability of a material depends on the polarity, crystallinity and glass transition temperature (Tg) of the material. Oxygen is a hydrophobic gas, therefore non-polar materials have higher oxygen solubility, and hence higher oxygen permeability than polar materials. Materials with low amount of crystallinity have higher oxygen permeability than material with high crystallinity. Therefore, materials with a low Tg is preferred.

Dimethyl silicone possesses the ability to allow various gases to permeate rapidly through it. This phenomenon is due primarily to the flexible silicone-oxygen-silicone linking sites of the silicone chain and an absence of crystallinity in silicone rubber. Technically speaking, the process of permeation through a non-porous material is actually a three stage activity. Whereas a porous material uses size exclusion as its method of separation, the process by which a non-porous membrane allows a permeation to occur is a much more complex means to an end. These steps are: sorption in, diffusion through, and desorption from the material by the permeating gas. The rate of permeation is the product of diffusivity and solubility coefficients of the permeating gas. The solubility coefficients for gases into dimethyl silicone are comparable to those of most polymers but the diffusion rates through the silicone are nearly an order of magnitude greater than any other membrane polymers. Therefore dimethyl silicone owes its rapid transport of gases to the high rate of diffusion and not solubility.

Preferably, the oxygen permeability of the material composing the hollow carrier is higher than $0.1 \cdot 10^{-7}$ ($cm^3/cm^2/cm/s$), higher than $1 \cdot 10^{-7}$ ($cm^3/cm^2/cm/s$), higher than $2 \cdot 10^{-7}$ ($cm^3/cm^2/cm/s$), more preferably higher than $5 \cdot 10^{-7}$ ($cm^3/cm^2/cm/s$), and even more preferably higher than $10 \cdot 10^{-7}$ ($cm^3/cm^2/cm/s$). These values represent the amount of oxygen that would permeate trough a 1 $cm^2$ specimen, 1 cm thick , in 1 second at 1 atm oxygen pressure. Accordingly, the oxygen permeability of the material composing the hollow carrier is higher than $0.1 \cdot 10^{-7}$ ($cm^3 \cdot cm/cm^2 \cdot s \cdot atm$), higher than $1 \cdot 10^{-7}$ ($cm^3 \cdot cm/cm^2 \cdot s \cdot atm$), higher than $2 \cdot 10^{-7}$ ($cm^3 \cdot cm/cm^2 \cdot s \cdot atm$), more preferably higher than $5 \cdot 10^{-7}$ ($cm^3 \cdot cm/cm^2 \cdot s \cdot atm$), and even more preferably higher than $10 \cdot 10^{-7}$ ($cm^3 \cdot cm/cm^2 \cdot s \cdot atm$).

Preferably, the glass transition temperature (Tg) of the material composing the hollow carrier is lower than 30°C, more preferably lower than 20°C, more preferably lower than 0°C, more preferably lower than -20°C, and even more preferably lower than -100°C.

The transport of oxygen through the walls of the hollow carrier will also be dependent on the thickness of the wall of the hollow carrier. The thickness of the walls of the hollow carrier is preferably less than 1 mm, less than 0.5 mm, preferably less than 0.2 mm, or even more preferably less than 0.1 mm.

In summary, the transport of oxygen per $cm^2$ through the walls of the hollow carrier is the product of the oxygen level of the provided oxygen containing gas, the pressure of the provided oxygen containing gas, the oxygen permeability of the material composing the hollow carrier, divided by the thickness of the walls of the hollow carrier.

As shown by the present inventors, the mechanical properties of the microbial cellulose vessels are dependent of the level of oxygen transport through the walls of the hollow carrier. Higher oxygen transport will result in microbial cellulose vessel with high mechanical strength.

It is essential that oxygen exchange from the inner side to the outer side of the hollow carrier is achieved through molecular diffusion of oxygen and not through the diffusion of micro-bubbles. Porous materials, like ceramic materials, woven materials, ePTFE, allow passage of micro-bubbles through the material of hollow carrier. The formation of bubbles on the outer side of the hollow carrier will disturb the formation of the microbial cellulose and will result in defects in the hollow microbial cellulose vessel formed.

Preferably, the hollow carrier is composed of a non-porous material. By a non-porous material is meant a material with no pores, no channels, and no rifts. Preferably, the hollow carrier is composed of a silicon polymer, such as dimethylsilicone, vinylmethyl silicone, fluorosilicone, diphenysilicone, or nitrile silicone. Silicone polymers are also designated polysiloxanes.

Preferably, the outer surface of the hollow carrier has a smooth surface with no pores and no rifts or other irregularities as the microbial formed on the outer surface of the hollow carrier will have an inner surface which is a replica of the outer surface of the hollow carrier.

Preferably, the method is performed by culturing cellulose-producing microorganisms on a branched hollow carrier leading to the production of a branched hollow microbial cellulose vessel.

There is provided a hollow cellulose vessel comprising cellulose produced by a microorganism. The hollow microbial cellulose vessel is produced by any of the methods of the present invention.

Further provided are hollow microbial cellulose vessels which are characterized by consisting of cellulose which is layered, where the layers are parallel to the walls of the vessel.

The hollow cellulose vessels are composed of microbial cellulose characterized by a high penetration resistance. The penetration resistance is preferably higher than 250 N/mm$^2$, higher than 300 N/mm$^2$, higher than 500 N/mm$^2$, and more preferably higher than 700 N/mm$^2$, such as higher than 1000 N/mm$^2$

The hollow microbial cellulose vessels are preferable in the form of microbial cellulose tubes.

Further provided is a tube essentially consisting of microbial cellulose. Preferably the microbial cellulose tube essentially consists of a microbial cellulose vessel.

The hollow microbial cellulose vessels can be of any dimension, linear, tapered and/or branched. The dimension and structure of the hollow microbial cellulose will be determined by the dimension and structure of the hollow carrier.

By using a hollow carrier which is branched, a microbial cellulose tube which is branched can be obtained. By using a hollow carrier which is tapered, a microbial cellulose tube which is tapered can be obtained.

The microbial cellulose tube is characterized by a high burst pressure. Preferably the burst pressure is higher than 100 mm Hg, higher than 150 mm Hg, higher than 250 mm Hg, higher than 300 mm Hg, higher than 400 mm Hg, higher than 500 mm Hg and more preferably higher than 800 mm Hg. The microbial cellulose tube is produced by the method of the present invention.

The microbial cellulose tube is composed of microbial cellulose characterized by a high penetration resistance. The penetration resistance is preferably higher than 250 N/mm$^2$, higher than 300 N/mm$^2$, higher than 500 N/mm$^2$, and more preferably higher than 700 N/mm$^2$, such as higher than 1000N /mm$^2$

The microbial cellulose vessels and the microbial cellulose tubes can be used in surgical procedures to replace a vessel in the animal or the human body, e.g. as an artificial blood vessel, urethra, ureter, trachea, a digestive tract vessel, or a lymphatic vessel.

The microbial cellulose vessels and the microbial cellulose tubes can be cut open and the formed patches of microbial cellulose used in surgical procedures to repair a vessel in the animal or the human body, e.g. a blood vessel, urethra, ureter, trachea, a digestive tract vessel, or a lymphatic vessel.

Provided is an artificial biological patch essentially consisting of a microbial cellulose vessel which has been cut open.

Provided is an artificial blood vessel which has a very good compatibility with a living body and superior mechanical properties. The artificial blood vessel is produced by a method of the present invention. The artificial blood vessel consists of a microbial cellulose tube.

In the present invention, any kind of cellulose producing microorganism can be used. For example, there can be mentioned Acetobacter xylinum, Acetobacter pasturianus, , Acetobacter aceti, Acetobacter ransens, Sarcina ventriculi, Bacterium xyloides, bacteria belonging to the genus Pseudomonas, bacteria belonging to the genus Agrobacterium, and bacteria belonging to Rhizobium. Preferably a strain of Acetobacter xylinum (also designated Gluconacetobacter xylinus) is used, such as, but not limited to, Acetobacter xylinum NCIB 8246 ATCC (American Type Culture Collection) number 23769,

Acetobacter xylinum NQ5 ATCC number 53582, or Acetobacter xylinum BPR2001 ATCC number 7000178

The cellulose is formed and accumulated by a usual bacterium-culturing method using a microorganism as mentioned above. Namely, the microorganism is added to a usual nutriment broth comprising a carbon source, a nitrogen source, inorganic salts and, if necessary, organic micronutrients such as amino acids and vitamins, and the culturing is conducted at a temperature of 20 to 40° C.

The method of the invention for preparing the hollow microbial vessels comprises culturing a cellulose producing microorganism on the outer surface of a hollow carrier. More specifically, the hollow carrier is immersed in a culture liquid, a cellulose-producing microorganism and a culture medium are supplied to the outer side of the hollow carrier, and the culturing is carried out by introducing an oxygen-containing gas on the inner side of the hollow carrier. If the culturing is conducted in this manner, a gelatinous cellulose having a thickness of 0.01 to 20 mm is formed on the surface of the carrier. The hollow carrier is removed and a hollow shaped article composed solely of the cellulose can be obtained.

Since the thus-prepared cellulose contains cells of the microorganism or culture medium ingredients, the cellulose can be washed as required, and this washing is carried out by using a dilute alkali, a dilute acid, an organic solvent and hot water, alone or in any combination thereof.

As the medium, there can be used polyhydric alcohols such as glycerol, erythritol, glycol, sorbitol and maltitol, saccharides such as glucose, galactose mannose, maltose and lactose, natural and synthetic high polymeric substances such as polyvinyl alcohol, polyvinyl pyrrolidone, polyethylene glycol, carboxymethyl cellulose, agar, starch, alginic acid salts, xanthane gum, polysaccharides, oligosaccharides, collagen, gelatin, and proteins, and water-soluble polar solvents such as acetonitrile dioxane, acetic acid, and propionic acid. These media can be used alone or in the form of a mixture or two or more thereof. Furthermore, a solution containing an appropriate solute can be used as the medium. Examples of suitable media are Schram Hestrin Media (Schramm et al. Biochem. J. 67 (1957) 669-679) (Glucose 20 g/l, Yeast extract 5 g/l, Peptone 5 g/l, $Na_2HPO_4$ 2.7 g/l, citric acid*$H_2O$ 1.15 g/l, pH 5), CSL media (Matsuoka et al. Biosci. Biotechn. Biochem. 60 (1996) 575-579) (fructose 40 g/l, $KH_2PO_4$ 1 g/l, $MgSO_4·7H_2O$ 0.25 g/l, $(NH_4)_2SO_4$ 3.3 g/l, vitamine mixture 1%, salt mixture 1%, CSL (corn steep liquor) 20 ml/l, lactate 0.15 %, pH 5-5.5), Son media (Son et al. Biotechnol. Appl. Biochem. 33 (2001) 1-5) (Glucose 15 g/l, $(NH_4)_2SO_4$ 2 g/l, $KH_2PO_4$ 3 g/l, $MgSO_4·7H_2O$ 0.8 g/l, $FeSO_4·7H_2O$ 5 mg/l, $H_3BO_3$ 3g/l, Nicotinamide 0.5 g/l, lactate 6%), or ATCC medium (Yeast extract 5 g/l, Peptone 3 g/l, Mannitol 25 g/l, agar 15 g/l).

Any pH between 3.5 and 6 is suited for practice of the method of the present invention.

The culture medium can be circulated and continuously replaced with fresh culture medium.

When the hollow microbial cellulose is used as an artificial blood vessel, the as-prepared hollow microbial cellulose can be directly substituted for a blood vessel in a living body, or the hollow microbial cellulose can be subjected to a certain preliminary treatment. For example, an adhesion of endothelial cells to the surface of the hollow microbial cellulose can be mentioned as the preliminary treatment.

The hollow microbial cellulose has a very good compatibility with a living body, especially blood, and has a high surface orienting property and a high mechanical strength.

LEGENDS TO FIGURES

Figure 1. Fermentation vessels.

A) (1) Gas inlet, (2) Inoculation tube, (3) Plug, (4) Hollow carrier composed of oxygen permeable material, (5) pyrex glass vessel, (6) culture media containing cellulose producing bacteria, (7) plug.
B) (1) Gas inlet and holder of hollow carrier, (2) Inoculation tube, (3) Gasket, (4) Hollow carrier composed of oxygen permeable material, (5) pyrex glass vessel or tube of stainless steel, (6) culture media.

Figure 2. Images of microbial cellulose tubes. A) long linear tube. B) branched tube.

C) Cross-sections of tubes with different diameters.

Figure 3. SEM (scanning electron microscopy) images of A) a microbial cellulose tube grown at 100% oxygen, B) a microbial cellulose tube grown at 35% oxygen.

Figure 4. SEM images of A) the inner surface and B) the outer surface of a microbial cellulose tube grown at 35% oxygen.

Figure 5. Cross sections of bacterial cellulose tubes seen with SEM. A) microbial cellulose tube grown at 20% oxygen, B) microbial cellulose tube grown at 35% oxygen, C) microbial cellulose tube grown at 50% oxygen; and D) microbial cellulose tube grown at 100% oxygen.

EXAMPLE 1

Fermentation

The fermentation of the tubes was carried out submerged in glass tubes of 70 ml by using a silicone tube (4x0.5 mm in diameter; 50 shores; Lebo production AB, Sweden) as oxygen permeable material. Gas mixtures with different concentrations of oxygen i.e. 21% (air), 35%, 50% and 100% at atmospheric pressure were provided into the oxygen-permeable. A complex media (CSL) (Matsuoka et al. Biosci., Biotechnol., Biochem. 60 (1996) 575-579) and a slightly modified defined media described by Son et al (Bioresource Technology 86 (2003) 215-219) were used as fermentation media. The Glucose and fructose consumption and pH were measured using standard enzymatic kit (R-Biopharm, Food Diagnostics AB Sweden). The strain used for the biosynthesis was Acetobacter xylinum subsp.sucrofermentas BPR2001, tradenmbr: 1700178™. The strain was purchased from the American Type Culture Collection. Six Cellulose forming colonies were cultivated for two days in Rough flask yielding a cell concentration of: $3.7·10^6$ cfu/ml. The bacteria were liberated from the resulting BC hydrogel by vigorous shaking and 2.5 ml was added to each fermentation vessel (Figure 1). The fermentations were completed after 5 days and the BC tubes and the hydrogel from the preculture were purified by boiling in 0.1 M NaOH, 60°C for 4 hours and thereafter repeated boiling in Millipore™ water. The BC tubes were steam sterilized by autoclaving for 20 minutes (120°C, 1bar) and stored in refrigerator until characterization and freeze-drying. The yield was recorded after drying the tubes in oven at 50°C until no weight change could be recorded.

The cellulose yield increases slightly by elevated oxygen ratio for the complex media, see Table I.

Table I. Yield of microbial cellulose obtained by culturing at different oxygen levels

| Oxygen ratio [%] | 21 | 35 | 50 | 100 |
|---|---|---|---|---|
| Yield Mean [mg] | 0,0377 | 0,0398 | 0,0451 | 0,0599 |
| Std.Err | 3,6744e-3 | 3,3829e-3 | 2,5560e-3 | 1,2865e-3 |

The yield at 100 % $O_2$ is significantly higher than for 20% and 35% $O_2$ respectively. This is contrary to the findings reported by Watanabe et al. (Biosci. Biotechnol. Biochem. 59 (1995) 65-68).

Morphology

*Scanning Electron Microscopy (SEM)*

SEM was used to study the morphology of the inner, the outer and section surfaces of the tubes. The material was froozen in liquid nitrogen before freeze-drying for 24h at -52°C, using a Heto PowerDry PL3000. The dried material was later coated with gold before the analysis, which was performed with a LEO 982 Gemini filed emission SEM.

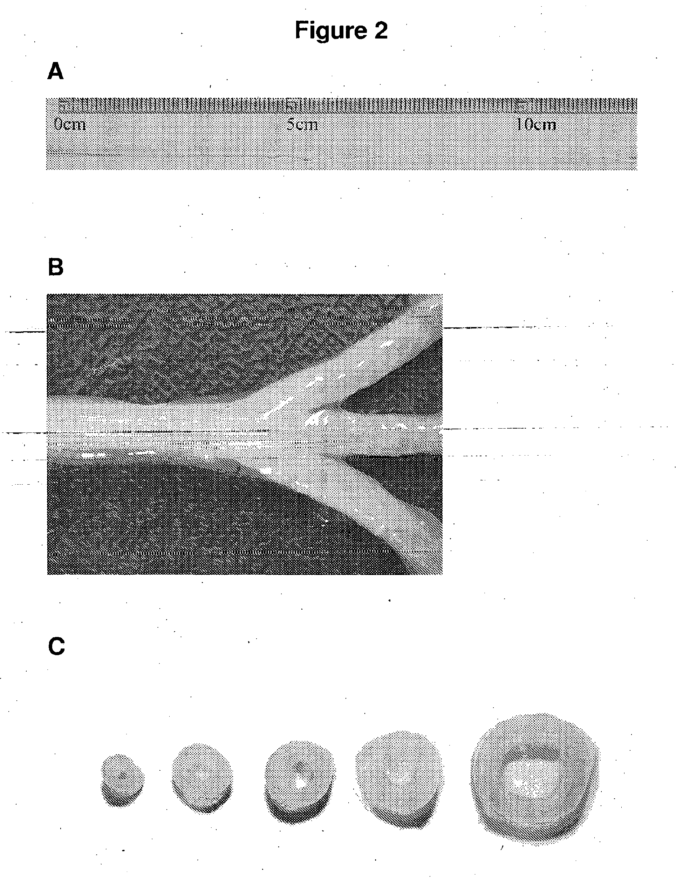

Images of microbial cellulose tubes can be seen in Figure 2. A change to a longer; narrower; wider; shorter silicone support subsequently gives a longer; narrower; wider; or shorter cellulose tube, respectively. There is consequently no limitation in length which otherwise is the case for the bacterial cellulose produced according to the static method reported by Klemm et al. (Progress in Polymer Science 26 (2001) 1561-1603; WO 01/61026). Moreover this fermentation technique is fast, it takes about seven days to produce a tube, as well as enables one to produce tapered and branched tubes.

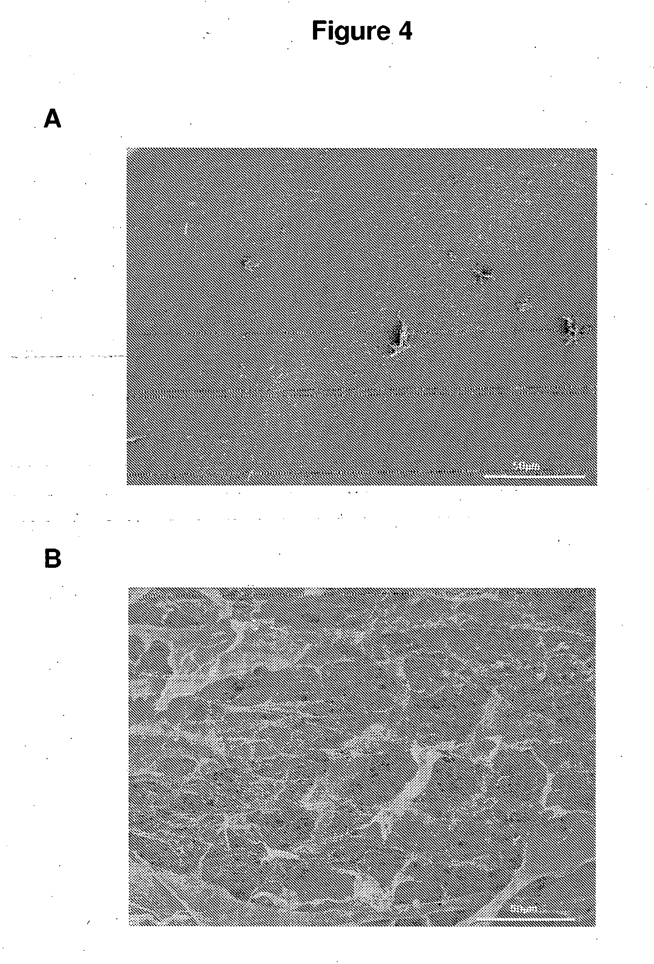

A SEM image of an inner and outer side of a bacterial cellulose tube can be seen in Figure 4. All the bacterial cellulose tubes have a smooth inner surface and a porous outer surface. Smoother surfaces have been shown to improve adhesion and proliferation of ECs and thus smooth inner surface of the tubes would be a favourable characteristics (Xu et al. J. Biomed. Mater. Res. Part A, 71 (2004) 154-161). A more open structure of the outer layer of the tube is advantageous for ingrowths of tissue and thus ingrowths of BC vessels into body.

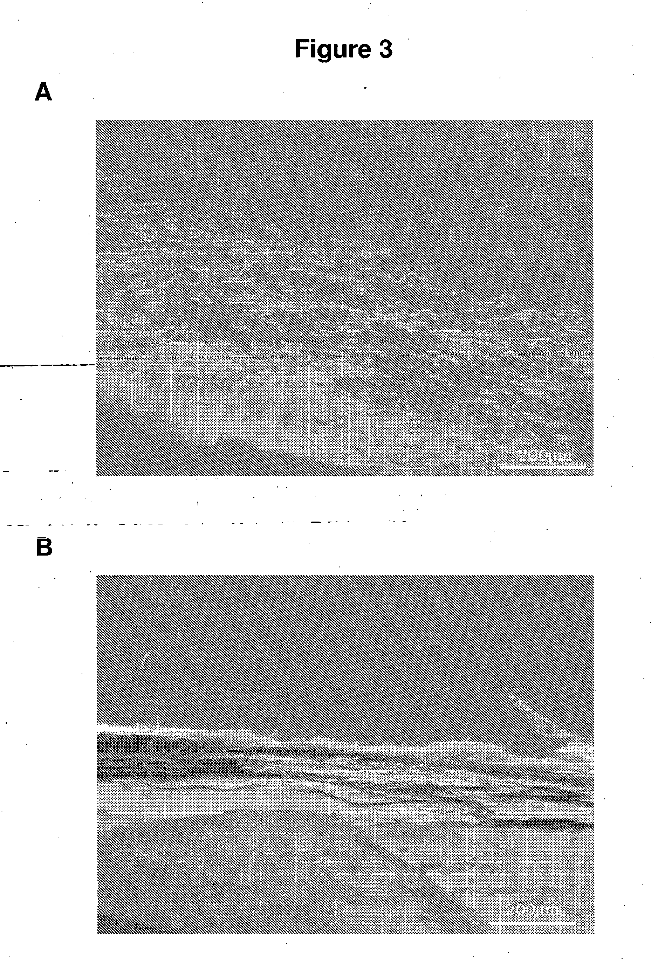

Cross section images of the tubes produced at the different oxygen levels show a layered structure, see Figure 3 and Figure 5. The thickness of the layered part varies with the level of oxygen in the provided gas. This part becomes thinner with decreased levels of oxygen, about 1.5 times thinner at 35% oxygen and nearly 2.5 times thinner at 20% oxygen, all compared to 100% oxygen. Figures 5A, B and D.

This can be compared with the horizontal layered structure obtained when growing bacterial cellulose static, as earlier described e.g by Klemm et al. (Prog. Polymer Sci. 26 (2001) 1561-1603). Most likely it is more favourable to have the layers parallel to the walls of the vessel as the strain induced by the blood flow is horizontal to the vessel wall.

EXAMPLE 2.

Mechanical properties

*Burst pressure measurement*

By applying a pressure through a water column, a flow with an increasing pressure was established. All tubes were tested on three locations upper, middle and lower part. The vessels were exposed to elevated pressure 1 bar/10 s. The pressure when burst occurred was registered.

*Texture and penetration resistance.*

The test was performed on a TA=XT2i Texture analyser (Stable Micro Systems, Surrey, England). The tube sample was cut open with a pair of scissors, in one cutting motion. The sample was placed over a hole in the sample holder and anchored tightly to the holder. A probe with a diameter of 2 mm was used and a load cell of 5 kg. Penetration force was measured at a speed of 0.1 mm/sec. The peak force was then recorded and compared with different materials. Tests were performed on tubes cultured with 50% oxygen and 100% oxygen; they had peak values of 1500N and 3800N respectively: Penetration resistance can be calculated to be 477 N/mm$^2$ and 1260 N/mm$^2$, respectively for each tested tube.

Results

Two different evaluations of the mechanical properties were done i.e. burst pressure and penetration resistance. The burst pressure results follow the same pattern as seen with the yield, see Table II.

Table II. Burst pressure of microbial cellulose tubes grown at different oxygen levels

| Oxygen ratio [%] | 21 | 35 | 50 | 100 |
|---|---|---|---|---|
| Burst pressure Mean [mmHg] | 300 | 517 | 709 | 885 |
| Std.Err | 0.0229 | 0.0528 | 0.0694 | 0.0687 |

Burst pressure measurements show clearly that the tubes get stronger i.e. burst at higher pressure with increasing oxygen levels. Tubes made at 21% and 35% oxygen are significantly different from tubes made at 50% and 100% oxygen.. The tubes sustain a blood pressure i.e. 250 mm Hg if produced at oxygen ratio over atmospheric and reach a top value of 880 mm Hg when produced at a ratio of 100% of oxygen. We propose that the inner layer with high density is required for the tube to sustain a certain pressure. Higher density of the inner layer at the air/media interface and an increase in layers by and increase in oxygen ratio might be an answer to the increasing burst pressure with increasing oxygen ratio.

EXAMPLE 3

Cell seeding

Endothelial cells (HSVECs) were isolated from healthy parts of human saphenous veins. Veins were either spare parts from coronary bypass operations or taken from patients having surgery for varicose vein. HSVECs were isolated using an enzymatic technique. The cells were seeded into the luminal side of the BC-tube and cultured under static conditions for 7 days humidified atmosphere of 95% air/5% $CO_2$ and a temperature of 37°C. Cells were fixed in 3.7% formaldehyde and the nuclei were counter stained with 4',6-diamidino-2-phenylindole,dihydrochloride, DAPI (Sigma-Aldrich).
Cultivation of endothelial cells onto the lumen of the bacterial cellulose tubes shows that we get a confluent layer of endothelial cells after 7 days.

**Claims**

1. A method for the preparation of hollow cellulose vessels by means of culturing cellulose-producing microorganisms on the outer surface of a hollow carrier, and providing an oxygen containing gas on the inner side of the hollow carrier, the method being **characterized by** the oxygen containing gas having an oxygen level in the range 35 % to 100 %.

2. The method according to claim 1 where the oxygen level is in the range 50 % to 100 %.

3. The method according to claim 2 where the oxygen level is in the range 80% to 100 %.

4. The method according to claim 3 where the oxygen level is 100 %.

5. The method according to any of claims 1 to 4 where the oxygen containing gas is provided at a pressure higher than atmospheric pressure.

6. The method according to any of claims 1 to 5, further **characterized by** the culturing being performed on a hollow carrier composed of a non-porous material with an oxygen permeability higher than $0.1\cdot10^{-7}$ ($cm^3\cdot cm/cm^2\cdot s\cdot atm$).

7. The method according to claim 6, wherein the non-porous material has an oxygen permeability higher than $1\cdot10^{-7}$ ($cm^3\cdot cm/cm^2\cdot s\cdot atm$).

8. The method according to claim 7, wherein the non-porous material has an oxygen permeability higher than $10\cdot10^{-7}$ ($cm^3\cdot cm/cm^2\cdot s\cdot atm$).

9. The method according to any of claims 1 to 8 further **characterized by** the culturing being performed on a hollow carrier composed of a material with a glass transition temperature lower than 30°C.

10. The method according to claim 9, wherein the glass transition temperature of said material is lower than 0°C.

11. The method according to claim 10, wherein the glass transition temperature of said material is lower than -20°C.

12. The method according to claim 11, wherein the glass transition temperature of said material is lower than -100°C.

13. The method according to any of claims 1 to 12, where the hollow carrier is positioned at a vertical position in the culture media.

14. The method according to any of claims 1 to 13, where the thickness of the walls of the hollow carrier is less than 1 mm.

15. The method according to claim 14, where the thickness of the walls of the hollow carrier is less than 0.5 mm.


**Patentansprüche**

1. Verfahren für die Herstellung hohler Zellulosegefäße mittels Kultivieren Zellulose produzierender Mikroorganismen auf der Außenoberfläche eines hohlen Trägers und Zuführen eines sauerstoffhaltigen Gases auf der Innenseite des hohlen Trägers, **dadurch gekennzeichnet, dass** das sauerstoffhaltige Gas einen Sauerstoffgehalt im Bereich 35-100 % aufweist.

2. Verfahren nach Anspruch 1, bei dem der Sauerstoffgehalt im Bereich 50-100 % liegt.

3. Verfahren nach Anspruch 2, bei dem der Sauerstoffgehalt im Bereich 80-100 % liegt.

4. Verfahren nach Anspruch 3, bei dem der Sauerstoffgehalt 100 % beträgt.

5. Verfahren nach einem der Ansprüche 1-4, bei dem das sauerstoffhaltige Gas bei einem Druck höher als Atmosphärendruck zugeführt wird.

6. Verfahren nach einem der Ansprüche 1-5, weiter **dadurch gekennzeichnet, dass** das Kultivieren durchgeführt wird auf einem hohlen Träger bestehend aus einem nicht porösen Material mit einer Sauerstoffdurchlässigkeit größer als $0,1 \cdot 10^{-7} (cm^3 \cdot cm/cm^2 \cdot s \cdot atm)$.

7. Verfahren nach Anspruch 6, bei dem das nicht poröse Material eine Sauerstoffdurchlässigkeit größer als $1 \cdot 10^{-7} (cm^3 \cdot cm/cm^2 \cdot s \cdot atm)$ aufweist.

8. Verfahren nach Anspruch 7, bei dem das nicht poröse Material eine Sauerstoffdurchlässigkeit größer als $10 \cdot 10^{-7} (cm^3 \cdot cm/cm^2 \cdot s \cdot atm)$ aufweist.

9. Verfahren nach einem der Ansprüche 1-8, weiter **dadurch gekennzeichnet, dass** das Kultivieren durchgeführt wird auf einem hohlen Träger bestehend aus einem Material mit einer Glasübergangstemperatur von weniger als 30 °C.

10. Verfahren nach Anspruch 9, bei dem die Glasübergangstemperatur des Materials geringer als 0 °C ist.

11. Verfahren nach Anspruch 10, bei dem die Glasübergangstemperatur des Materials geringer als -20 °C ist.

12. Verfahren nach Anspruch 11, bei dem die Glasübergangstemperatur des Materials geringer als -100 °C ist.

13. Verfahren nach einem der Ansprüche 1-12, bei dem der hohle Träger in einer vertikalen Stellung in dem Kulturmedium positioniert ist.

14. Verfahren nach einem der Ansprüche 1-13, dem die Dicke der Wände des hohlen Trägers weniger als 1 mm beträgt.

**15.** Verfahren nach Anspruch 14, bei dem die Dicke der Wände des hohlen Trägers weniger als 0,5 mm beträgt.

**Revendications**

**1.** Procédé pour la préparation de vaisseaux de cellulose creux au moyen de la culture de micro-organismes produisant de la cellulose sur la surface extérieure d'un support creux, et la fourniture d'un gaz contenant de l'oxygène du côté intérieur du support creux, le procédé étant **caractérisé par** le gaz contenant de l'oxygène ayant une teneur en oxygène dans la plage de 35 % à 100 %.

**2.** Procédé selon la revendication 1, où la teneur en oxygène est dans la plage de 50 % à 100 %.

**3.** Procédé selon la revendication 2, où la teneur en oxygène est dans la plage de 80 % à 100 %.

**4.** Procédé selon la revendication 3, où la teneur en oxygène est de 100 %.

**5.** Procédé selon l'une quelconque des revendications 1 à 4, où le gaz contenant de l'oxygène est alimenté à une pression supérieure à la pression atmosphérique.

**6.** Procédé selon l'une quelconque des revendications 1 à 5, **caractérisé en outre par** la culture étant réalisée sur un support creux composé d'un matériau non poreux avec une perméabilité à l'oxygène supérieure à $0,1.10^{-7}$ ($cm^3.cm/cm^2.s.atm$).

**7.** Procédé selon la revendication 6, dans lequel le matériau non poreux a une perméabilité à l'oxygène supérieure à $1.0^{-7}$ ($cm^3.cm/cm^2.s.atm$).

**8.** Procédé selon la revendication 7, dans lequel le matériau non poreux a une perméabilité à l'oxygène supérieure à $10.10^{-7}$ ($cm^3.cm/cm^2.s.atm$).

**9.** Procédé selon l'une quelconque des revendications 1 à 8, **caractérisé en outre par** la culture étant effectuée sur un support creux composé d'un matériau ayant une température de transition vitreuse inférieure à 30°C.

**10.** Procédé selon la revendication 9, dans lequel la température de transition vitreuse dudit matériau est inférieure à 0°C.

**11.** Procédé selon la revendication 10, dans lequel la température de transition vitreuse dudit matériau est inférieure à -20°C.

**12.** Procédé selon la revendication 11, dans lequel la température de transition vitreuse dudit matériau est inférieure à -100°C.

**13.** Procédé selon l'une quelconque des revendications 1 à 12, où le support creux est positionné dans une position verticale dans le milieu de culture.

**14.** Procédé selon l'une quelconque des revendications 1 à 13, où l'épaisseur des parois du support creux est inférieure à 1 mm.

**15.** Procédé selon la revendication 14, où l'épaisseur des parois du support creux est inférieure à 0,5 mm.

# Figure 1

# Figure 2

**A**

**B**

**C**

# Figure 3

A

B

# Figure 4

A

B

# Figure 5

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3165774 A
- JP 8126697 A
- EP 186495 A2
- JP 63205109 A
- JP 3272772 A
- EP 396344 A2
- WO 0161026 A1
- WO 8912107 A
- US 6017740 A
- EP 0792935 A
- WO 2007027849 A
- WO 0161026 A

**Non-patent literature cited in the description**

- **KLEMM et al.** *Prog. Polymer Sci.,* 2001, vol. 26, 1561-1603
- Manufacturing and characterization of bacterial cellulose tubes using two different fermentation techniques. **BODIN et al.** MODERN MULTIDISCIPLINARY APPLIED MICROBIOLOGY. WILEY-VCH, July 2006, 619-622
- **SCHRAMM ; HESTRIN.** *J. Gen. Microbiol.,* 1954, vol. 11, 123-129
- **WATANABE et al.** *Biosci. Biotechnol. Biochem.,* 1995, vol. 59, 65-68
- *Prog. Polymer Sci.,* 2001, vol. 26, 1561-1603
- **SCHRAMM et al.** *Biochem. J.,* 1957, vol. 67, 669-679
- **MATSUOKA et al.** *Biosci. Biotechn. Biochem.,* 1996, vol. 60, 575-579
- **SON et al.** *Biotechnol. Appl. Biochem.,* 2001, vol. 33, 1-5
- **MATSUOKA et al.** *Biosci., Biotechnol., Biochem,* 1996, vol. 60, 575-579
- **SON et al.** *Bioresource Technology,* 2003, vol. 86, 215-219
- **WATANABE et al.** *Biosci. Biotechnol. Biochem,* 1995, vol. 59, 65-68
- **KLEMM et al.** *Progress in Polymer Science,* 2001, vol. 26, 1561-1603
- **XU et al.** *J. Biomed. Mater. Res. Part A,* 2004, vol. 71, 154-161